# EUROPEAN PATENT APPLICATION

(11) **EP 4 410 765 A1**
(43) Date of publication of application: **07.08.2024**
(21) Application number: 22875619.3
(22) Date of filing: 10.08.2022
(51) Int. Cl.: C07C 17/25, B01J 27/132, C07C 17/20, C07C 21/18, C07B 61/00

(54) **METHOD FOR PRODUCING (E)-1,1,1,4,4,4-HEXAFLUORO-2-BUTENE**

(30) Priority: 30.09.2021 JP 2021160792
(71) Applicant: Resonac Corporation, Tokyo 105-7325 (JP)
(72) Inventor: YAMAGAMI Motoya, Tokyo 105-8518 (JP); KOBAYASHI Hiroshi, Tokyo 105-8518 (JP); SUZUKI Atsushi, Tokyo 105-8518 (JP)
(74) Representative: Strehl Schübel-Hopf & Partner
(86) International application number: PCT/JP2022/030667
(87) International publication number: WO 2023/053744

(57) **Abstract**

Provided is a method capable of producing (E)-1,1,1,4,4,4-hexafluoro-2-butene at a high yield. The method for producing (E)-1,1,1,4,4,4-hexafluoro-2-butene includes vaporizing 1,2,3,4-tetrachlorobutane and reacting the 1,2,3,4-tetrachlorobutane vaporized in the vaporizing, with chlorine gas and hydrogen fluoride gas in the gas phase in the presence of a solid catalyst to yield (E)-1,1,1,4,4,4-hexafluoro-2-butene.

## Description

### Technical Field

The present invention relates to a method for producing (E)-1,1,1,4,4,4-hexafluoro-2-butene.

### Background Art

PTL 1 discloses a technology of producing (E)-1,1,1,4,4,4-hexafluoro-2-butene useful as an etching gas for microfabrication of semiconductors, by reacting butane, butene, or butadiene with chlorine gas and hydrogen fluoride.

### Citation List

### Patent Literature

PTL 1: JP 6-228023 A

### Summary of Invention

### Technical Problem

By the technology disclosed in PTL 1, however, 1,1,1,4,4,4-hexafluoro-2,3-dichloro-2-butene and 1,1,1,4,4,4-hexafluoro-2-chloro-2-butene are formed together with 1,1,1,4,4,4-hexafluoro-2-butene, and the selectivity of 1,1,1,4,4,4-hexafluoro-2-butene is about 20% of them. Hence, the technology has a problem of a low yield of 1,1,1,4,4,4-hexafluoro-2-butene.

The present invention is intended to provide a method capable of producing (E)-1,1,1,4,4,4-hexafluoro-2-butene at a high yield.

### Solution to Problem

To solve the problems, aspects of the present invention are the following [1] to [5].
[1] A method for producing (E)-1,1,1,4,4,4-hexafluoro-2-butene, the method including
   vaporizing 1,2,3,4-tetrachlorobutane, and
   reacting the 1,2,3,4-tetrachlorobutane vaporized in the vaporizing, with chlorine gas and hydrogen fluoride gas in the gas phase in the presence of a solid catalyst to yield (E)-1,1,1,4,4,4-hexafluoro-2-butene.
[2] The method for producing (E)-1,1,1,4,4,4-hexafluoro-2-butene according to the aspect [1], in which the solid catalyst is a hydrogen fluoride-treated material prepared by bringing hydrogen fluoride gas into contact with an oxide of at least one metal selected from silver, cobalt, aluminum, chromium, manganese, nickel, zinc, iron, and indium under a condition of 190°C or more and 400°C or less.
[3] The method for producing (E)-1,1,1,4,4,4-hexafluoro-2-butene according to the aspect [1] or [2], in which in the reacting, the ratio of the molar amount of the chlorine gas subjected to the reaction to the molar amount of the 1,2,3,4-tetrachlorobutane subjected to the reaction is 2 or more and 10 or less, and the ratio of the molar amount of the hydrogen fluoride gas subjected to the reaction to the molar amount of the 1,2,3,4-tetrachlorobutane subjected to the reaction is 5 or more and 50 or less.
[4] The method for producing (E)-1,1,1,4,4,4-hexafluoro-2-butene according to any one of the aspects [1] to [3], in which in the vaporizing, the 1,2,3,4-tetrachlorobutane is vaporized in a vaporizer filled with a filler.
[5] The method for producing (E)-1,1,1,4,4,4-hexafluoro-2-butene according to any one of the aspects [1] to [4], in which in the vaporizing, the 1,2,3,4-tetrachlorobutane is vaporized in the presence of at least one dilution gas selected from chlorine gas, nitrogen gas, helium gas, and argon gas.

### Advantageous Effects of Invention

According to the present invention, (E)-1,1,1,4,4,4-hexafluoro-2-butene can be produced at a high yield.

### Description of Embodiments

Embodiments of the present invention will now be described. The embodiments are merely examples of the present invention, and the present invention is not limited to the embodiments. Various modifications or improvements can be made in the embodiments, and such modifications and improvements can be encompassed by the present invention.

The inventors of the present invention have studied the production of (E)-1,1,1,4,4,4-hexafluoro-2-butene from various chlorinated butanes as materials, have found the materials and production conditions with which (E)-1,1,1,4,4,4-hexafluoro-2-butene can be produced at a high yield, and have completed the present invention. The present invention relates to a method for producing (E)-1,1,1,4,4,4-hexafluoro-2-butene. The method uses 1,2,3,4-tetrachlorobutane (hereinafter also called "TCB") as the material and enables the production of (E)-1,1,1,4,4,4-hexafluoro-2-butene at a high yield.

In other words, the method for producing (E)-1,1,1,4,4,4-hexafluoro-2-butene pertaining to the present embodiment includes vaporizing 1,2,3,4-tetrachlorobutane and reacting the 1,2,3,4-tetrachlorobutane vaporized in the vaporizing, with chlorine gas (Cl₂) and hydrogen fluoride gas (HF) in the gas phase in the presence of a solid catalyst to yield (E)-1,1,1,4,4,4-hexafluoro-2-butene.

Hereinafter, the method for producing (E)-1,1,1,4,4,4-hexafluoro-2-butene pertaining to the present embodiment will be described in detail.

Chlorinating TCB with chlorine gas under heating yields a chlorinated butane in which each terminal carbon atom of the butane skeleton is completely chlorinated as described by Reaction Scheme (i). Simultaneously with the chlorination, dehydrochlorination also proceeds to yield a chlorinated butene in which each terminal carbon atom of the butene skeleton is completely chlorinated as described by Reaction Scheme (ii).

CClH₂-CClH-CClH-CClH₂ + nCl₂ → CCl₃-CX1Y1-CX2Y2-CCl₃ + nHCl Reaction Scheme (i)

CClH₂-CClH-CClH-CClH₂ + nCl₂ → CCl₃-CX1=CY1-CCl₃ + (n + 1)HCl Reaction Scheme (ii)

In Reaction Schemes (i) and (ii), X1, X2, Y1, and Y2 are each independently a chlorine atom or a hydrogen atom.

The chlorinated butane or the chlorinated butene in which each terminal carbon atom is completely chlorinated easily reacts with hydrogen fluoride gas on a solid catalyst to undergo halogen exchange together with dehydrohalogenation, and hexafluoro butene in which each terminal of the butene skeleton is completely fluorinated is formed.

The inventors of the present invention have found that when TCB is used as a material to produce the chlorinated butane or the chlorinated butene in which each terminal carbon atom is completely chlorinated as described above, by-products having similar chemical structures are also formed but the yield (selectivity) of the chlorinated butane or the chlorinated butene in which each terminal carbon atom is completely chlorinated is higher than that when butane or butene is used as a material.

The inventors of the present invention have also found that when TCB is reacted with chlorine gas and hydrogen fluoride gas in the gas phase in the presence of a solid catalyst, the yield of (E)-1,1,1,4,4,4-hexafluoro-2-butene is extremely higher when the reaction is performed in a reaction field to which a gaseous TCB, chlorine gas, and hydrogen fluoride gas are fed than that when the reaction is performed in a reaction field to which a liquid or solid TCB, chlorine gas, and hydrogen fluoride gas are fed (i.e., when vaporizing TCB is not included).

The inventors of the present invention have further found that in the vaporizing, 1,2,3,4-tetrachlorobutane is preferably vaporized in the presence of a dilution gas. In other words, in the vaporizing, when a dilution gas and TCB are previously mixed and the TCB coexistent with the dilution gas is vaporized, TCB is vaporized more efficiently than when TCB is vaporized without any dilution gas. Mixing with a dilution gas further improves the mixing state of the vaporized TCB with chlorine gas and hydrogen fluoride gas (hereinafter, each of chlorine gas and hydrogen fluoride gas may also be called a "reaction gas"), and thus TCB is unlikely to undergo thermal decomposition on a solid catalyst. This improves the conversion rate of TCB to increase the yield of (E)-1,1,1,4,4,4-hexafluoro-2-butene. Examples of the dilution gas suitable to improve the mixing state of TCB and the reaction gas include chlorine gas and inert gases and more specifically include at least one gas selected from chlorine gas, nitrogen gas (N₂), helium gas (He), and argon gas (Ar).

When the vaporizing is performed by using a vaporizer and the reacting is performed by using a reactor, a liquid or solid TCB is heated and vaporized in the vaporizer to give a gaseous TCB, and the gaseous TCB is fed to the reactor. Only the TCB may be fed to the vaporizer for vaporization, the TCB together with a dilution gas may be fed to the vaporizer as described above for vaporization of the TCB, or the TCB together with hydrogen fluoride may be fed to the vaporizer for vaporization of the TCB.

The vaporizing temperature of TCB in the vaporizing is required to be a temperature at which TCB can be vaporized at a pressure in the vaporizing. As long as TCB can be vaporized, the vaporizing temperature and the vaporizing pressure are not limited, but TCB has a boiling point of about 190°C under atmospheric pressure, and thus the vaporizing temperature is preferably 190°C or more when the vaporizing is performed under atmospheric pressure.

An excessively high vaporizing temperature, however, may cause thermal decomposition of TCB. Hence, the vaporizing temperature is preferably 190°C or more and 450°C or less, more preferably 200°C or more and 400°C or less, and even more preferably 200°C or more and 300°C or less.

To improve the vaporizing efficiency of a liquid TCB in the vaporizing, the vaporizer is preferably filled with a filler for increasing the vaporizing area of TCB. As the material of the filler, an inert metal not causing TCB to deteriorate, ceramics, or the like may be used. Examples of the metal used as the filler include nickel (Ni), iron (Fe), copper (Cu), zinc (Zn), and aluminum (Al). Examples of the ceramic include alumina, silica, and zeolite. The filler may have a pellet shape, a spherical shape, a Raschig shape, a mesh shape, or the like, and, for example, a metal mesh may be used as the filler.

The reaction of TCB with chlorine gas and hydrogen fluoride gas (chlorination with chlorine gas and fluorination with hydrogen fluoride gas) is required to be performed by gas phase reaction. Accordingly, the reaction temperature is required to be a temperature at which TCB can exist as a gas. As long as gas phase reaction can be performed, the reaction temperature and the reaction pressure are not limited, but TCB has a boiling point of about 190°C under atmospheric pressure, and thus the reaction temperature is preferably 190°C or more when the reacting is performed under atmospheric pressure.

An excessively high reaction temperature, however, may cause thermal decomposition of TCB, and the solid catalyst may be covered with carbides. This may result in a lower catalytic activity. Accordingly, the reaction temperature is preferably 190°C or more and 400°C or less, more preferably 200°C or more and 350°C or less, and even more preferably 200°C or more and 300°C or less.

When the reacting is performed by using a reactor and the temperature of the reactor is controlled by heating from the outside, the temperature of the whole reactor may be controlled. Alternatively, the reactor may be divided into a plurality of portions, and the temperature of each portion may be controlled. For example, control may be performed such that the temperature near the inlet of a reactor is higher than the temperature near the outlet.

In the method for producing (E)-1,1,1,4,4,4-hexafluoro-2-butene pertaining to the present embodiment, chlorination with chlorine gas and fluorination with hydrogen fluoride gas are performed in the reacting. In the reacting, the chlorination and the fluorination may be performed in the same reactor or may be performed in separate reactors. To produce (E)-1,1,1,4,4,4-hexafluoro-2-butene by fluorination with hydrogen fluoride gas, however, a chlorinated butane in which each terminal carbon atom of the butane skeleton is completely chlorinated is required to be used, and thus chlorination of TCB is required to be performed before fluorination with hydrogen fluoride gas.

When, for example, two reactors are prepared, chlorination and fluorination can be separately performed in different reactors, and thus the temperatures of the reactors can be set at different temperatures. This enables more appropriate setting of the reaction temperature of each reaction. When chlorination and fluorination are separately performed in different reactors, however, the former chlorination yields an intermediate having a high boiling point, and thus the reaction temperature of the latter fluorination is preferably set at a temperature 50 to 100°C higher than the reaction temperature of the chlorination.

When chlorination and fluorination are performed in the same reactor, the chlorination and the fluorination can be performed in a single reactor, and this has an advantage from the viewpoint of equipment costs. When chlorination and fluorination are performed in the same reactor, a compound formed by the chlorination of TCB undergoes the fluorination for a short period of time, and thus the product of chlorination and fluorination has a boiling point not more than that of TCB. Hence, the reaction temperature can be set in a wider range than when chlorination and fluorination are separately performed in different reactors. At a low reaction temperature, the solid catalyst can be used for a longer time.

When chlorination and fluorination are performed in the same reactor, however, the chlorination and the fluorination are performed with the same catalyst at the same reaction temperature. The conditions are thus slightly unfavorable from the viewpoint of performing the chlorination and the fluorination under the respective optimum conditions. In order not to form by-products or not to facilitate carbonization of by-products having high boiling points, the solid catalysts described below are preferably used.

When chlorination and fluorination are performed in the same reactor, the solid catalyst is preferably an oxide of at least one metal selected from silver (Ag), cobalt (Co), aluminum (Al), chromium (Cr), manganese (Mn), nickel (Ni), zinc (Zn), iron (Fe), and indium (In) or a fluorinated product of the oxide, which has an excellent activity in various catalyst forms. As the metal oxide, a catalyst containing aluminum oxide is preferred, and a catalyst containing aluminum oxide and chromium oxide is more preferred. Aluminum oxide can also be used as a support or a supported substance and functions as a catalyst in each case.

For example, the solid catalyst may be such a supported type catalyst that the above metal oxide or a fluorinated product of the oxide is used as the support to support another substance, is supported on a support of another substance, or is used both as the support and the supported substance. Alternatively, the solid catalyst may be a catalyst prepared by tableting a mixture of the above metal oxide or a fluorinated product of the oxide with a binder.

When chlorination and fluorination are separately performed in different reactors, a chlorinated product of the above metal oxide is preferably used as the solid catalyst for chlorination of TCB, and a fluorinated product of the oxide is preferably used as the solid catalyst for fluorination of the chlorinated TCB.

The solid catalyst may be a catalyst in the form of a supported type containing the above metal oxide or a chlorinated or fluorinated product of the above metal oxide or may be a catalyst prepared by tableting a mixture of the above metal oxide or a chlorinated or fluorinated product of the above metal oxide with a binder.

As an example of the solid catalyst, a supported type catalyst will be described. As the support, for example, aluminum oxide (Al₂O₃, grade NST-3 manufactured by NIKKI-UNIVERSAL) may be used. The support is impregnated with an aqueous chromium chloride (CrCls) solution, and the whole is dried at 100 to 120°C and then burned in air at 380 to 420°C to give chromium oxide (Cr₂O₃). The product is brought into contact with hydrogen gas (H₂) at a temperature of 380 to 420°C to reduce the excessively oxidized chromium, and a catalyst that supports chromium oxide as the supported substance is produced. The supported amount of chromium oxide relative to the total catalyst amount of aluminum oxide as the support and the supported chromium oxide is, for example, 10% by mass or more and 20% by mass or less.

Next, as another example of the solid catalyst, a tableted type catalyst will be described. An aqueous chromium nitrate (Cr(NO₃)₃) solution is neutralized with aqueous ammonia (NH₃) to precipitate chromium hydroxide (Cr(OH)₃) particles, and the neutralized liquid is filtered to separate chromium hydroxide. The chromium hydroxide is next washed with water to remove nitrate ions or ammonium ions and is dried in air at 100 to 120°C. The resulting dried product is pulverized, and a binder such as graphite is added. The mixture is tableted by using a tableting machine.

The formed pellets are packed into a burning chamber and are burned in air at 380 to 420°C, and chromium hydroxide is converted into chromium oxide (Cr₂O₃). The product is then brought into contact with hydrogen gas at a temperature of 380 to 420°C, and the excessively oxidized chromium is reduced to yield a tableted type chromium oxide catalyst. To use an additional metal other than chromium together, a nitrate of the additional metal may be mixed with chromium nitrate as the material.

As the solid catalyst used in the above chlorination fluorination or the fluorination in the reacting, a hydrogen fluoride-treated material prepared by bringing hydrogen fluoride gas into contact with an oxide of at least one metal selected from silver, cobalt, aluminum, chromium, manganese, nickel, zinc, iron, and indium may be used as the fluorinated catalyst. The hydrogen fluoride-treated material can be in various forms and is difficult to define by compositions or structures.

As the solid catalyst used in the above chlorination in the reacting, a chlorine-treated material prepared by bringing chlorine gas into contact with an oxide of at least one metal selected from silver, cobalt, aluminum, chromium, manganese, nickel, zinc, iron, and indium may be used as the chlorinated catalyst. The chlorine-treated material can be in various forms and is difficult to define by compositions or structures.

When a reactor is used to perform the reacting, an oxide of at least one metal selected from silver, cobalt, aluminum, chromium, manganese, nickel, zinc, iron, and indium may be packed into the reactor before the reacting, and hydrogen fluoride gas or chlorine gas may be brought into contact with the above metal oxide at a high temperature by introducing the hydrogen fluoride gas or chlorine gas into the reactor. Accordingly, the treatment of giving a hydrogen fluoride-treated material or a chlorine-treated material by treating the above metal oxide with hydrogen fluoride gas or chlorine gas can be performed in the reactor. Then, the reacting can be successively performed in the reactor by using the hydrogen fluoride-treated material or the chlorine-treated material as the solid catalyst.

The treatment temperature of the hydrogen fluoride treatment and the chlorine treatment is preferably 190°C or more and 400°C or less, more preferably 300°C or more and 400°C or less, and even more preferably 340°C or more and 400°C or less under atmospheric pressure in order to efficiently yield the hydrogen fluoride-treated material and the chlorine-treated material. The pressure in the hydrogen fluoride treatment and the chlorine treatment may be, for example, 0.01 MPa or more and 0.5 MPa or less in terms of absolute pressure but is preferably substantially atmospheric pressure.

When the hydrogen fluoride treatment is performed in a reactor, the material of the reactor preferably has corrosion resistance against hydrogen fluoride gas because the reactor into which hydrogen fluoride gas is introduced comes into contact with hydrogen fluoride gas and water as a by-product. Preferred examples of the material include metals such as Inconel (registered trademark) and Hastelloy (registered trademark).

When the chlorine treatment is performed in a reactor, the material of the reactor into which chlorine gas is introduced preferably has corrosion resistance against chlorine gas and hydrogen chloride gas (HCl). Preferred examples of the material include metals such as stainless steel SUS304 and ceramics such as quartz glass. The above material having corrosion resistance against hydrogen fluoride gas is also usable as a material of the reactor when the chlorine treatment is performed in the reactor.

It is thought that by previously performing hydrogen fluoride treatment or chlorine treatment of a metal oxide before the reacting, the reactivity variation of a catalyst caused by fluorination with hydrogen fluoride gas or chlorination with chlorine gas can be suppressed and the reaction is easily controlled at a constant rate. When a metal oxide is previously subjected to hydrogen fluoride treatment or chlorine treatment, the reaction controllability is improved as compared with when hydrogen fluoride treatment or chlorine treatment is not performed, and this has an advantage of suppressing formation of by-products or carbonization of by-products having high boiling points.

In the reacting, the amounts of TCB, chlorine gas, and hydrogen fluoride gas subjected to the reaction are preferably set at the following values. In other words, the ratio of the molar amount of chlorine gas subjected to the reaction to the molar amount of TCB subjected to the reaction is preferably 2 or more and 10 or less, more preferably 2 or more and 6 or less, and even more preferably 2 or more and 4 or less.

The ratio of the molar amount of hydrogen fluoride gas subjected to the reaction to the molar amount of TCB subjected to the reaction is preferably 5 or more and 50 or less, more preferably 5 or more and 20 or less, and even more preferably 5 or more and 10 or less.

When the above two ratios are within the above numerical ranges, the yield of (E)-1,1,1,4,4,4-hexafluoro-2-butene is likely to be higher.

The total amount of the gases subjected to the reaction (for example, the total amount of the gases fed to a reactor) is preferably 50 (/h) or more and 1,000 (/h) or less, more preferably 50 (/h) or more and 300 (/h) or less, and even more preferably 100 (/h) or more and 300 (/h) or less in terms of space velocity (SV) calculated with gas under the standard condition.

At a space velocity (SV) of 50 (/h) or more, the reactor for the reacting does not have an excessively large volume and is economically advantageous. At a space velocity (SV) of 1,000 (/h) or less, the yield of (E)-1,1,1,4,4,4-hexafluoro-2-butene is likely to be higher.

In the reacting, the outlet gas discharged from the outlet of the reactor may be purified in the following manner. In other words, unreacted chlorine gas, unreacted hydrogen fluoride, and hydrogen chloride as a by-product may be removed from the outlet gas, and the resulting organic compound may be purified by distillation or adsorption. For example, the difference in boiling point between an E-isomer (boiling point: 8°C) and a Z-isomer (boiling point: 33°C) can be used to separate the isomers by a common distillation operation.

Such purification can yield highly pure (E)-1,1,1,4,4,4-hexafluoro-2-butene. Unreacted chlorine gas, unreacted hydrogen fluoride, and hydrogen chloride as a by-product may be removed by using a boiling point difference from the organic compound or may be removed by washing the outlet gas with an alkaline water or the like.

### Examples

The present invention will next be described more specifically with reference to examples and comparative examples, but the invention is not limited to the examples.

### [Example 1]

A vaporizer for performing the vaporization step of vaporizing 1,2,3,4-tetrachlorobutane (TCB) was prepared. Separately, a reactor for performing the reaction step of reacting TCB with chlorine gas and hydrogen fluoride gas in the gas phase in the presence of a solid catalyst to yield (E)-1,1,1,4,4,4-hexafluoro-2-butene was prepared.

The vaporizer was made of stainless steel SUS316, had a tubular shape having an inner diameter of 20 mm and a length of 120 mm, and was filled with a metal mesh made of nickel as the filler. The reactor was made of Inconel (registered trademark), had a tubular shape having an inner diameter of 1 inch and a length of 170 mm, and was filled with a solid catalyst. The preparation method of the solid catalyst will first be described.

Aluminum oxide (Al₂O₃, grade NST-3 manufactured by NIKKI-UNIVERSAL, an average particle size of 3 mm) that supported chromium oxide (Cr₂O₃) was prepared. The supported amount of chromium oxide was 21% by mass relative to the total mass of the aluminum oxide catalyst supporting chromium oxide. Next, 53 g (100 mL, a bulk density of 0.53 g/mL) of the aluminum oxide supporting chromium oxide was packed into the reactor, and the temperature in the reactor was increased to 340°C while nitrogen gas was allowed to flow through the reactor. Then, hydrogen fluoride gas was fed under atmospheric pressure such that the temperature in the reactor was maintained at 400°C or less, and hydrogen fluoride gas was brought into contact with the aluminum oxide supporting chromium oxide to yield a hydrogen fluoride-treated material of the aluminum oxide supporting chromium oxide. By such a treatment, a hydrogen fluoride-treated solid catalyst (in Table 1, denoted by "solid catalyst A") was prepared in the reactor.

Next, the vaporizing step and the reacting step were performed by using the vaporizer and the reactor to produce (E)-1,1,1,4,4,4-hexafluoro-2-butene. First, from a stainless steel SUS316 syringe filled with a liquid 1,2,3,4-tetrachlorobutane (a molecular weight of 196 g/mol), TCB was fed by using a syringe pump at a speed of 0.094 g/min to the vaporizer. To the vaporizer, chlorine gas as the dilution gas was simultaneously fed (at a gas flow rate of 25 mL/min).

The temperature in the vaporizer was controlled at 250°C under atmospheric pressure, and the fed liquid TCB was vaporized in the vaporizer to give a TCB gas. In the vaporizer, the TCB gas and the chlorine gas as the dilution gas were mixed to give a mixed gas, and the mixed gas was taken out of the vaporizer and fed to the reactor.

To the reactor, together with the mixed gas of TCB gas and chlorine gas, chlorine gas and hydrogen fluoride gas as the reaction gases and nitrogen gas for dilution were fed. The chlorine gas in the mixed gas fed from the vaporizer also functioned as the reaction gas. The gas flow rate of the chlorine gas fed to the reactor (except the chlorine gas in the mixed gas) was 10 mL/min, the gas flow rate of the hydrogen fluoride gas was 173 mL/min, and the gas flow rate of the nitrogen gas was 25 mL/min. Accordingly, the feed rate of the whole gas to the reactor was 250 mL/min, and the space velocity (SV) was 250 × 60/100 = 150/h. The molar ratio of chlorine gas to TCB was 2, and the molar ratio of hydrogen fluoride gas to TCB was 10.

While the temperature in the reactor (i.e., the temperature of the solid catalyst) was controlled at 250°C, TCB, chlorine gas, and hydrogen fluoride gas were reacted to cause chlorination and fluorination, and (E)-1,1,1,4,4,4-hexafluoro-2-butene was formed. The outlet gas discharged from the outlet of the reactor was fed to a trap of an aqueous potassium hydroxide (KOH) solution, and the outlet gas was washed. The outlet gas after washing was then analyzed by using a gas chromatograph-mass spectrometer, and the compounds contained in the outlet gas were identified and quantified. The results are described in Table 1. The detector of the gas chromatograph was a hydrogen flame ionization detector (FID). Measurement conditions of the gas chromatography are as described below.
Gas chromatograph: GC-2014 manufactured by Shimadzu Corporation
Column: DB-1
Injection temperature: 200°C
Column temperature: maintained at 40°C for 15 minutes → increased at a temperature increase rate of 10°C/min to 230°C → maintained at 230°C for 32 minutes
Detector: FID
Detector temperature: 230°C
Carrier gas: helium
Detection limit: 1 ppm by mass

The outlet gas contained, in addition to the target compound (E)-1,1,1,4,4,4-hexafluoro-2-butene (in Table 1, denoted by "E-isomer"), compounds as described below: (Z)-1,1,1,4,4,4-hexafluoro-2-butene (in Table 1, denoted by "Z-isomer"; 2-chloro-1,1,1,4,4,4-hexafluoro-2-butene (in Table 1, denoted by "monochloro product"); and 2,3-dichloro-1,1,1,4,4,4-hexafluoro-2-butene (in Table 1, denoted by "dichloro product").

The outlet gas further contained, in addition to the above E-isomer, the Z-isomer, the monochloro product, and the dichloro product, compounds as described below: 2-chloro-1,1,1,3,4,4,4-heptafluoro-2-butene; 2,3-dichloro-1,1,1,4,4-pentafluoro-2-butene; 1,2,3-trichloro-1,1,4,4,4-pentafluoro-2-butene; 1,2-dichloro-1,1,4,4,4-pentafluoro-2-butene; 1,2,4-trichloro-1,1,4,4-tetrafluoro-2-butene; and the like (in Table 1, these compounds are collectively denoted by "others").

**[Table 1]**

| | Solid catalyst | Temperature in vaporizer and reactor (°C) | Yield (% by mole) | | | | |
|---|---|---|---|---|---|---|---|
| | | | E-isomer | Z-isomer | Monochloro product | Dichloro poroduct | Others |
| Ex. 1 | A | 250 | 35.5 | 0.6 | 9.5 | 2.3 | 52.1 |
| Ex. 2 | A | 280 | 40.6 | 0.7 | 26.7 | 4.3 | 27.7 |
| Ex. 3 | A | 350 | 53.3 | 0.7 | 23.2 | 2.5 | 20.3 |
| Ex. 4 | B | 250 | 46.7 | 0.5 | 14.2 | 2.5 | 36.1 |
| Ex. 5 | B | 200 | 53.3 | 0.7 | 23.2 | 2.5 | 20.3 |
| Ex. 6 | B | 280 | 53.5 | 0.7 | 23.3 | 2.5 | 20.0 |
| Ex. 7 | B | 350 | 41.1 | 1.0 | 18.4 | 2.5 | 37.0 |
| Ex. 8 | A | 250 | 31.4 | 0.8 | 7.3 | 1.9 | 58.6 |
| Ex. 9 | A | 250 | 30.3 | 0.5 | 6.4 | 1.3 | 61.5 |
| Ex. 10 | A | 250 | 34.2 | 0.7 | 10.4 | 2.5 | 52.2 |
| Ex. 11 | A | 400 | 55.4 | 1.0 | 20.3 | 1.2 | 22.1 |
| Comp. Ex. 1 | A | 250 | 7.2 | 0.2 | 4.5 | 0.4 | 87.7 |
| Comp. Ex. 2 | A | 250 | 4.8 | 0.2 | 3.1 | 0.3 | 91.6 |
| Comp. Ex. 3 | Without | 250 | less than 0.1 | less than 0.1 | 0.2 | 0.5 | 99.3 |
| Comp. Ex. 4 | A | 180 | 0.8 | less than 0.1 | 0.5 | 0.3 | 98.4 |

### [Example 2]

(E)-1,1,1,4,4,4-Hexafluoro-2-butene was produced in the same manner as in Example 1 except that the temperature in the vaporizer and the reactor was 280°C. The results are described in Table 1.

### [Example 3]

(E)-1,1,1,4,4,4-Hexafluoro-2-butene was produced in the same manner as in Example 1 except that the temperature in the vaporizer and the reactor was 350°C. The results are described in Table 1.

### [Example 4]

Chromium oxide (Cr₂O₃) powder, aluminum oxide (Al₂O₃) powder, and indium oxide (In₂O₃) powder were mixed and tableted to give tablets. As for the proportion of each powder, chromium oxide was 89% by mass, aluminum oxide was 5% by mass, and indium oxide was 6% by mass.

Into a reactor substantially the same as in Example 1, 95 g of the resulting tablets (100 mL, a bulk density of 0.95 g/mL) were packed, and the temperature in the reactor was increased to 350 to 380°C while nitrogen gas was allowed to frow through the reactor. Hydrogen fluoride gas was then fed under atmospheric pressure such that the temperature in the reactor was maintained at 400°C or less, and the hydrogen fluoride gas was brought into contact with the tablets to yield hydrogen fluoride-treated materials of the chromium oxide, the aluminum oxide, and the indium oxide. By such hydrogen fluoride treatment, a solid catalyst of the hydrogen fluoride-treated materials (in Table 1, denoted by "solid catalyst B") was prepared in the reactor. (E)-1,1,1,4,4,4-Hexafluoro-2-butene was produced in the same manner as in Example 1 except that solid catalyst B was used as the catalyst. The results are described in Table 1.

### [Example 5]

(E)-1,1,1,4,4,4-Hexafluoro-2-butene was produced in the same manner as in Example 4 except that the temperature in the vaporizer and the reactor was 200°C. The results are described in Table 1.

### [Example 6]

(E)-1,1,1,4,4,4-Hexafluoro-2-butene was produced in the same manner as in Example 4 except that the temperature in the vaporizer and the reactor was 280°C. The results are described in Table 1.

### [Example 7]

(E)-1,1,1,4,4,4-Hexafluoro-2-butene was produced in the same manner as in Example 4 except that the temperature in the vaporizer and the reactor was 350°C. The results are described in Table 1.

### [Example 8]

(E)-1,1,1,4,4,4-Hexafluoro-2-butene was produced in the same manner as in Example 1 except that chlorine gas as the dilution gas was not fed at all but only TCB was fed to the vaporizer in the vaporizing step, and chlorine gas was fed to the reactor only in the reacting step. The results are described in Table 1. No chlorine gas was fed to the vaporizer in the vaporizing step, and thus the gas flow rate of chlorine gas fed to the reactor in the reacting step was increased to 35 mL/min. The space velocity (SV) of all the gases fed to the reactor was 150/h as with Example 1. The molar ratio of chlorine gas to TCB was 2, and the molar ratio of hydrogen fluoride gas to TCB was 10.

### [Example 9]

(E)-1,1,1,4,4,4-Hexafluoro-2-butene was produced in the same manner as in Example 1 except that no filler was packed into the vaporizer. The results are described in Table 1.

### [Example 10]

(E)-1,1,1,4,4,4-Hexafluoro-2-butene was produced in the same manner as in Example 1 except that nitrogen gas was fed as a dilution gas in place of chlorine gas to the vaporizer under the same conditions in the vaporizing step, and the gas flow rate of chlorine gas fed in the reacting step was changed to 35 mL/min. The space velocity (SV) of all the gases fed to the reactor was 150/h as with Example 1. As with Example 1, the molar ratio of chlorine gas to TCB was 2, and the molar ratio of hydrogen fluoride gas to TCB was 10. The results are described in Table 1.

### [Example 11]

(E)-1,1,1,4,4,4-Hexafluoro-2-butene was produced in the same manner as in Example 1 except that the temperature in the vaporizer and the reactor was 400°C. The results are described in Table 1.

### [Comparative Example 1]

(E)-1,1,1,4,4,4-Hexafluoro-2-butene was produced in the same manner as in Example 1 except that butane (C₄H₁₀) was used as the material in place of TCB at a gas flow rate of 17 mL/min. The space velocity (SV) of all the gases fed to the reactor was 150/h as with Example 1. The molar ratio of chlorine gas to butane was 2, and the molar ratio of hydrogen fluoride gas to butane was 10. The results are described in Table 1.

### [Comparative Example 2]

(E)-1,1,1,4,4,4-Hexafluoro-2-butene was produced in the same manner as in Example 1 except that no vaporizing step was performed but TCB was fed by directly feeding a liquid TCB to the reactor. The results are described in Table 1.

The liquid TCB was fed by using a syringe pump at a flow rate of 0.094 g/min. The gas flow rate of the chlorine gas fed to the reactor was 35 mL/min, the gas flow rate of the hydrogen fluoride gas was 173 mL/min, and the gas flow rate of the nitrogen gas was 25 mL/min. The space velocity (SV) of all the gases fed to the reactor was 140/h, the molar ratio of chlorine gas to TCB was 2, and the molar ratio of hydrogen fluoride gas to TCB was 10.

### [Comparative Example 3]

(E)-1,1,1,4,4,4-Hexafluoro-2-butene was produced in the same manner as in Example 1 except that no solid catalyst was packed at all into the reactor. The results are described in Table 1.

### [Comparative Example 4]

(E)-1,1,1,4,4,4-Hexafluoro-2-butene was produced in the same manner as in Example 1 except that the temperature in the vaporizer and the reactor was 180°C, which was less than the boiling point of TCB. In other words, (E)-1,1,1,4,4,4-hexafluoro-2-butene was produced in the same manner as in Example 1 except that the reaction of TCB with chlorine gas and hydrogen fluoride gas was not performed in the gas phase. The results are described in Table 1.

### [Reference Example 1]

Reaction was performed in the same manner as in Example 1 except that nitrogen gas for dilution was fed at the same flow rate to the reactor in place of hydrogen fluoride gas. In other words, no fluorination with hydrogen fluoride gas was performed, but only chlorination of TCB with chlorine gas was performed.

As a result, TCB was reacted with chlorine gas to yield butanes or butenes in which each terminal carbon atom was completely chlorinated at a high selectivity. In other words, 1,1,1,2,3,4,4,4-octachlorobutane, 1,1,1,2,3,4,4,4-octachloro-2-butene, and 1,1,1,2,4,4,4-heptachloro-2-butene were produced, and the total yield was about 75% by mole.

In addition, butanes or butenes in which each terminal carbon atom was not completely chlorinated were produced. In other words, 1,1,1,2,3,4,4-heptachlorobutane, 1,1,1,2,3,4,4-heptachloro-2-butene, and 1,1,2,3,4,4-hexachloro-2-butene were produced, and the total yield was about 20% by mole.

### [Reference Example 2]

Reaction was performed in the same manner as in Example 1 except that nitrogen gas for dilution was fed at the same flow rate to the reactor in place of hydrogen fluoride gas and butane was used as the material in place of TCB. In other words, no fluorination with hydrogen fluoride gas was performed, but only the chlorination of butane with chlorine gas was performed.

As a result, butane was reacted with chlorine gas to yield butanes or butenes in which each terminal carbon atom is completely chlorinated at a low selectivity. In other words, 1, 1, 1,2, 3, 4, 4, 4-octachlorobutane, 1,1,1,2,3,4,4,4-octachloro-2-butene, and 1,1,1,2,4,4,4-heptachloro-2-butene were produced, and the total yield was about 45% by mole.

In addition, butanes or butenes in which each terminal carbon atom was not completely chlorinated were produced. In other words, 1,1,1,2,3,4,4-heptachlorobutane, 1,1,1,2,3,4,4-heptachloro-2-butene, and 1,1,2,3,4,4-hexachloro-2-butene were produced, and the total yield was about 50% by mole.

### [Reference Example 3]

Reaction was performed in the same manner as in Example 1 except that nitrogen gas for dilution was fed at the same flow rate to the reactor in place of hydrogen fluoride gas and 2-butene (C₄H₈) was used as the material in place of TCB. In other words, no fluorination with hydrogen fluoride gas was performed, but only the chlorination of 2-butene with chlorine gas was performed.

As a result, 2-butene was reacted with chlorine gas to yield butanes or butenes in which each terminal carbon atom was completely chlorinated at a low selectivity. In other words, 1, 1, 1,2, 3, 4, 4, 4-octachlorobutane, 1,1,1,2,3,4,4,4-octachloro-2-butene, and 1,1,1,2,4,4,4-heptachloro-2-butene were produced, and the total yield was about 38% by mole.

In addition, butanes or butenes in which each terminal carbon atom was not completely chlorinated were produced. In other words, 1,1,1,2,3,4,4-heptachlorobutane, 1,1,1,2,3,4,4-heptachloro-2-butene, and 1,1,2,3,4,4-hexachloro-2-butene were produced, and the total yield was about 56% by mole.

### [Reference Example 4]

Reaction was performed in the same manner as in Example 1 except that nitrogen gas for dilution was fed at the same flow rate to the reactor in place of hydrogen fluoride gas and butadiene (C₄H₆) was used as the material in place of TCB. In other words, no fluorination with hydrogen fluoride gas was performed, but only the chlorination of butadiene with chlorine gas was performed.

As a result, butadiene was reacted with chlorine gas to yield butanes or butenes in which each terminal carbon atom was completely chlorinated at a low selectivity. In other words, 1, 1, 1,2, 3, 4, 4, 4-octachlorobutane, 1,1,1,2,3,4,4,4-octachloro-2-butene, and 1,1,1,2,4,4,4-heptachloro-2-butene were produced, and the total yield was about 43% by mole.

In addition, butanes or butenes in which each terminal carbon atom was not completely chlorinated were produced. In other words, 1,1,1,2,3,4,4-heptachlorobutane, 1,1,1,2,3,4,4-heptachloro-2-butene, and 1,1,2,3,4,4-hexachloro-2-butene were produced, and the total yield was about 52% by mole.

Reference Examples 1, 2, 3, and 4 revealed the following findings. In other words, in the case of Reference Example 1 in which the catalyst pertaining to the present invention was used to chlorinate TCB, butanes or butenes in which each terminal carbon atom was completely chlorinated were produced at a high selectivity. However, when butane, 2-butene, or butadiene was used in place of TCB as in Reference Examples 2, 3, and 4, butanes or butenes in which each terminal carbon atom was completely chlorinated were produced at a lower selectivity. In the present invention, using TCB as the starting material is advantageous.

## Claims

1. A method for producing (E)-1,1,1,4,4,4-hexafluoro-2-butene, the method comprising:
vaporizing 1,2,3,4-tetrachlorobutane; and
reacting the 1,2,3,4-tetrachlorobutane vaporized in the vaporizing, with chlorine gas and hydrogen fluoride gas in a gas phase in a presence of a solid catalyst to yield (E)-1,1,1,4,4,4-hexafluoro-2-butene.

2. The method for producing (E)-1,1,1,4,4,4-hexafluoro-2-butene according to claim 1, wherein the solid catalyst is a hydrogen fluoride-treated material prepared by bringing hydrogen fluoride gas into contact with an oxide of at least one metal selected from silver, cobalt, aluminum, chromium, manganese, nickel, zinc, iron, and indium under a condition of 190°C or more and 400°C or less.

3. The method for producing (E)-1,1,1,4,4,4-hexafluoro-2-butene according to claim 1 or claim 2, wherein in the reacting, a ratio of a molar amount of the chlorine gas subjected to the reaction to a molar amount of the 1,2,3,4-tetrachlorobutane subjected to the reaction is 2 or more and 10 or less, and a ratio of a molar amount of the hydrogen fluoride gas subjected to the reaction to the molar amount of the 1,2,3,4-tetrachlorobutane subjected to the reaction is 5 or more and 50 or less.

4. The method for producing (E)-1,1,1,4,4,4-hexafluoro-2-butene according to any one of claims 1 to 3, wherein in the vaporizing, the 1,2,3,4-tetrachlorobutane is vaporized in a vaporizer filled with a filler.

5. The method for producing (E)-1,1,1,4,4,4-hexafluoro-2-butene according to any one of claims 1 to 4, wherein in the vaporizing, the 1,2,3,4-tetrachlorobutane is vaporized in a presence of at least one dilution gas selected from chlorine gas, nitrogen gas, helium gas, and argon gas.
